# FASCICULE DE BREVET EUROPEEN

(11) **EP 2 879 759 B1**
(45) Date de publication et mention de la délivrance du brevet: **09.10.2019**
(21) Numéro de dépôt: 13774058.5
(22) Date de dépôt: 06.08.2013
(51) Int. Cl.: A61N 1/39

(54) **DISPOSITIF DE TRAITEMENT DE DONNEES COMPORTANT UN DEFIBRILLATEUR ET DEFIBRILLATEUR COMMUNICANT**
DATENVERARBEITUNG MIT EINEM DEFIBRILLATOR UND KOMMUNIZIERENDER DEFIBRILLATOR
DATA PROCESSING DEVICE COMPRISING A DEFIBRILLATOR AND COMMUNICATING DEFIBRILLATOR

(30) Priorité: 06.08.2012 FR 1202189
(43) Date de publication de la demande: 10.06.2015
(73) Titulaire: Cadet, Pierre-Henri, 06400 Cannes (FR); Ricard, Philippe, 06200 Nice (FR)
(72) Inventeur: Cadet, Pierre-Henri, 06400 Cannes (FR); Ricard, Philippe, 06200 Nice (FR)
(74) Mandataire: Schuffenecker, Thierry
(86) Numéro de dépôt international: PCT/EP2013/002354
(87) Numéro de publication internationale: WO 2014/023422

(56) Documents cités:
- EP-A2- 1 834 622
- WO-A1-03/101537
- WO-A2-2008/057302
- US-A1- 2008 306 562
- US-A1- 2009 240 297
- US-B1- 8 081 071

## Description

### Domaine technique de l'invention

La présente invention concerne le milieu médical et notamment le domaine des défibrillateurs externes.

### Etat de la technique

Les statistiques du ministère de la Santé font état de 50000 décès chaque année dus à des accidents cardio-respiratoires. Dans presque la moitié des cas, ces accidents sont provoqués par une fibrillation ventriculaire initiale qui, en l'absence d'un traitement adéquat appliqué dans les deux ou trois minutes, entraîne la mort.

Heureusement, l'on dispose aujourd'hui de défibrillateurs qui permettent, lorsque l'on intervient dans ce délai de deux à trois minutes, de délivrer un choc et, par conséquent, de rétablir le fonctionnement normal du coeur. Ces défibrillateurs qu'il s'agisse de défibrillateurs internes ou externes, se sont généralisés ces dernières années, sortant du domaine hospitalier et du milieu des professionnels de santé pour s'insinuer dans le monde de travail et se répandre dans le grand public.

Les accidents cardio-respiratoires peuvent en effet apparaître n'importe où et frapper n'importe qui, que celui-ci se trouve dans son environnement professionnel, en train de faire des courses, sur le lieu de ses loisirs ou au restaurant.

Lorsque intervient l'accident, l'urgence est immédiate et, bien évidemment, les médecins et les urgentistes sont à même de prodiguer des soins précis et diligents et d'effectuer les gestes indispensables pour la mise en oeuvre d'un défibrillateur.

La demande WO-A-2008/057302 décrit un dispositif défibrillateur externe automatisé comportant des fonctionnalité de monitoring sans fil.

La demande US-A-2009/0240297 décrit un défibrillateur externe susceptible de fonctionner en coopération avec un téléphone mobile ;

La demande EP-A-1834622 décrit un dispositif de défibrillation automatisé avec un capteur de ventilation.

La demande US-A-2008/0306562 décrit un dispositif médical comportant un ensemble d'électrodes et capteurs destinés à l'obtention d'une réponse d'un patient.

La demande WO-A-03/101537 décrit une dispositif permettant la capture d'images d'une scène de défibrillation.

Le brevet US-B-8081071 décrit un système et un procédé de monitoring de dispositif médicaux portables externes.

### Exposé de l'invention

La présente invention a pour but de proposer un nouveau dispositif de défibrillateur plus accessible pour les usagers non professionnels et le grand public en général, susceptible de conduire à une plus grande efficacité.

Un autre but de la présente invention consiste à proposer un dispositif de défibrillation susceptible d'une plus grande simplicité de mise en oeuvre pour le grand public et ce en tout lieu où est susceptible de frapper l'accident cardio-respiratoire.

C'est un autre but de la présente invention que de renforcer la sécurité de l'utilisation d'un défibrillateur pour un premier secours qui n'est pas un professionnel de la Santé publique.

L'invention réalise ces buts au moyen d'un dispositif mobile de traitement de données, tel qu'un ordinateur portable, une tablette tactile ou un téléphone intelligent, comportant, de fabrication :
- un circuit électronique de défibrillation ;
- une batterie destinée à alimenter ledit circuit de défibrillation ;
- un logiciel d'assistance à l'utilisation pour permettre l'utilisation du défibrillateur par le grand public.

Le dispositif comporte une centrale de diagnostique, monitoring médical, comportant au moins un capteur permettant de suivre une donnée physiologique de l'utilisateur du dispositif.

En particulier, l'on dispose un capteur infrarouge destiné à la saisie de la saturation en oxygène et de la fréquence cardiaque de l'utilisateur du dispositif mobile , ainsi que son enregistrement périodique au sein d'une zone de stockage contrôlée par le logiciel de diagnostique.

De préférence, le capteur infra-rouge est localisé à proximité immédiate d'un capteur d'empreinte digitale présent à des fins de sécurité, de manière à assurer conjointement :
- la saisie de la courbe de pression et de fréquence cardiaque en même temps que la prise d'empreinte digitale destinée à l'activation du système ;
- la signature des courbes de pression et de fréquence cardiaque par une information dérivée du capteur d'empreinte digitale de manière à associer irréversiblement les données saisies par le capteur infrarouge et l'empreinte digitale de l'utilisateur

Dans un mode de réalisation particulier, le dispositif comporte deux électrodes permettant la capture d'un électrocardiogramme dans une configuration bipolaire et :
- des moyens de stockage de l'électrocardiogramme au sein du dispositif;
- des moyens d'analyse de l'électrocardiogramme et de comparaison avec une image de référence ;
- en réponse à ladite comparaison, des moyens d'alerte de l'utilisateur du dispositif

Le présente description divulgue également un procédé de diagnostique automatique pour un dispositif mobile tel que défini précédemment, comprenant les étape :
- la capture d'un électrocardiogramme de référence, simultanément à une donnée d'identification de l'utilisateur ;
- l'association de l'image de référence avec des données caractéristiques de l'utilisateur,
- la transmission de l'image de référence à un serveur ou à un système distant de qualification ;
- réception d'une confirmation de qualification de l'image de référence ;
- stockage et utilisation de l'image de référence à des fins de comparaison avec des nouveaux électrocardiogrammes capturés par ledit système mobile.

En particulier, l'on peut avantageusement associer l'ECG capturé grâce au dispositif lors de la mise en route du système avec une empreinte digitale également saisie (au moyen d'un même doigt) lors de cette mise en route. L'on parvient ainsi à sécuriser significativement la qualification d'image ECG de référence qui servira ensuite ultérieurement lors de l'utilisation du système.

La présente description divulgue également un dispositif défibrillateur comportant un circuit électronique de défibrillation, des électrodes ainsi qu'une batterie autonome. Le dispositif comporte des moyens de communication avec un système de traitement de l'information mobile comportant des moyens d'affichage et/ou audio, de manière à permettre le déroulement d'un assistant multimédia lors d'une intervention de défibrillation.

La proximité immédiate que l'on crée ainsi entre un défibrillateur - objet complexe et de maniement délicat - et ces nouveaux moyens techniques qui foisonnent dans l'environnement immédiat de tout un chacun, permet d'accroître significativement l'impact de l'intervention. En outre, en bénéficiant des fonctions multimédias de ces mêmes systèmes d'information, l'on sécurise grandement le geste effectué par un intervenant non professionnel.

Dans un premier mode de réalisation particulier, le dispositif défibrillateur comporte des moyens de communication avec un téléphone mobile de type intelligent (*smartphone* dans la littérature anglo-saxonne ou encore *Portable Digital Assistant*), comportant notamment un système d'exploitation et doté de fonctionnalités multimédias, de manière à permettre l'accès aux fonctionnalités multimédias dudit téléphone. Ces moyens de communication pourront être notamment de type WIFI ou *Bluetooth*

De préférence, le dispositif comporte notamment l'installation sur le téléphone intelligent, d'un logiciel permettant le contrôle épisodique du niveau de charge de la batterie autonome.

Dans un second mode de réalisation particulier, le dispositif défibrillateur comporte des moyens de communication avec une tablette tactile mobile, de manière à permettre l'accès aux fonctionnalités multimédias de ladite tablette.

De préférence également, le dispositif comporte des moyens de communication avec la tablette tactile permettant le contrôle épisodique du niveau de charge de la batterie.

Dans un mode de réalisation particulier, le dispositif est logé dans un accessoire de ladite tablette, à savoir une housse, un étui ou une coque de cette tablette. Plus spécifiquement, le dispositif défibrillateur est logé dans un compartiment scellé de manière à ne permettre un unique accès aux électrodes de défibrillation, accroissant ainsi la sécurité de son fonctionnement. L'on assure ainsi la sécurité de la fonction en évitant qu'un défibrillateur ayant déjà servi puisse être réutilisé par un opérateur.

Dans un mode de réalisation particulier, le dispositif défibrillateur comporte des moyens de détection de l'état de scellement du compartiment d'accès aux électrodes, de manière à commander l'affichage d'un signal avertisseur sur l'écran de ladite tablette.

Dans un mode de réalisation particulier, le dispositif défibrillateur comporte des moyens de communication avec un ordinateur, tel qu'un ordinateur portable ou ultra-portable de manière à permettre un accès à des fonctionnalités multimédia dudit ordinateur.

De préférence, le dispositif se présente sous la forme d'un kit batterie comportant en son sein les circuits de défibrillations et la batterie autonome et pouvant se substituer à une batterie d'origine du fabricant.

Dans un mode de réalisation particulier, le dispositif défibrillateur comporte des moyens permettant d'organiser un double fonctionnement avec ledit ordinateur:
- un premier fonctionnement dans le cadre d'un système d'exploitation natif présent au sein de l'ordinateur, destiné à la mise en oeuvre de procédures non critiques de maintenance du système de défibrillation;
- un second fonctionnement forçant l'extinction du système d'exploitation du système d'exploitation préexistant au sein de l'ordinateur, de manière à réserver l'intégralité des ressources au dispositif de défibrillation.

L'on accroît significativement la sécurité de l'utilisation du défibrillateur dans une situation d'urgence.

Dans un mode de réalisation particulier, le dispositif défibrillateur comporte un connecteur de type USB permettant d'être utilisé pour un démarrage à froid de l'ordinateur.

Dans un autre mode de réalisation particulier, le défibrillateur comporte:
- des moyens permettant de détecter le branchement des électrodes de défibrillation;
- des moyens de commande, en réponse à la détection dudit branchement, pour:
- forcer l'extinction du système d'exploitation en réponse à la détection du branchement des électrodes de défibrillation;
- activer le connecteur USB de manière à permettre un démarrage à froid (*boot*) immédiat via ledit connecteur USB.

Dans un autre mode de réalisation particulier le défibrillateur prend la forme d'un "*caddie*" destiné à remplacer le module de lecture de CD/DVD de manière à rendre accessible en permanence un défibrillateur.

Enfin, la présente divulgation concerne la réalisation d'un ordinateur, tel qu'un ordinateur portable doté de toutes les fonctionnalités d'un défibrillateur.

### Description des dessins

D'autres caractéristiques, but et avantages de la présente divulgation apparaîtront à la lecture de la description et des dessins ci-après, donnés uniquement à titre d'exemples non limitatifs. Sur les dessins annexés :
La figure 1 illustre un premier mode de réalisation d'un défibrillateur adapté à la coopération avec un dispositif de traitement de données de type téléphone mobile.
La figure 2 illustre un second mode de réalisation d'un défibrillateur adapté à la coopération avec un dispositif de traitement de données de type tablette tactile.
La figure 3 illustre un troisième mode de réalisation d'un défibrillateur adapté à la coopération avec un ordinateur portable ou ultra-portable, sous la forme d'un Kit-batterie spécifique.
La figure 4 illustre un quatrième mode de réalisation d'un défibrillateur adapté à la coopération avec un ordinateur portable ou ultra-portable, sous la forme d'un caddie spécifique destiné à remplacer le module de lecteur de CD-DVD.
La figure 5 illustre un cinquième mode de réalisation se présentant sous la forme d'un système de traitement mobile de l'information, tel qu'un ordinateur portable ou une tablette comportant, de fabrication un défibrillateur doté, le cas échéant, d'un logiciel de diagnostique médical.
La figure 6 illustre un mode de réalisation préféré d'un procédé de diagnostique automatique mis en oeuvre dans le cinquième mode de réalisation.

### Description des modes de réalisation préférés

L'on va décrire à présent comment l'on peut accroître significativement l'impact d'un défibrillateur en lui permettant une communication adaptée avec l'un des nombreux systèmes de traitement d'information - notamment mobiles - qui composent notre environnement, qu'il s'agisse des ordinateurs portables, des téléphones mobiles, tablettes tactiles, des assistants numériques personnels (*Portable Digital assistant* dans la littérature anglo-saxonne) etc... qui foisonnent dans notre environnement immédiat.

De cette manière, l'on accroît significativement la possibilité pour tout un chacun de trouver, dans une situation d'urgence où l'espérance de vie se mesure en moins d'une dizaine de minutes, un dispositif de défibrillation complet capable d'être mis en oeuvre d'une manière simple et ce notamment par un opérateur non professionnel qui ne dispose pas des compétences, des connaissances et du sang froid des professionnels de la santé.

A cet égard, afin d'accroître la simplicité de la solution préconisée, l'on propose la mise en oeuvre d'un dispositif défibrillateur susceptible de communiquer de manière adéquate avec un des nombreux systèmes d'information mobiles présent dans l'environnement courant, de manière à accéder - notamment - aux fonctionnalités multimédias de ces mêmes systèmes.

En permettant à un opérateur - par hypothèse non professionnel - de bénéficier des fonctionnalités multimédias d'un système d'information mobile, qu'il s'agisse d'un ordinateur portable, d'une tablette tactile, d'un téléphone intelligent (désigné dans la littérature anglo-saxonne sous le vocable "*smartphone*"), d'un assistant numérique personnel (PDA) l'on accroît ainsi considérablement non seulement la **DISPONIBILITE** de ces défibrillateurs mais également leur **SIMPLICITE** d'utilisation et, par conséquent, les perspectives de survie d'une victime potentielle d'un accident cardio-respiratoire.

Tout en préservant, comme on va le voir plus loin, la **SECURITE** de l'opération, grâce aux multiples aménagements qui seront décrits ci-après dans les différents modes de réalisation.

Pour illustrer de multiples possibilités d'adaptation, l'on va à présent décrire cinq modes de réalisation particuliers , lesquels ne sont d'ailleurs pas exclusifs l'un par rapport à l'autre.
- un défibrillateur doté de moyens de communication avec un dispositif de traitement de données de type téléphone mobile, ou assistant portable (PDA);
- un défibrillateur doté de moyens de communication avec une tablette tactile;
- un défibrillateur doté de moyens de communication avec un ordinateur portable, prenant la forme d'un kit "batterie";
- un défibrillateur doté de moyens de communication avec un ordinateur portable, prenant la forme d'un "caddie".
- un système de traitement de l'information mobile comportant, de fabrication, un dispositif défibrillateur complet et doté, fort avantageusement dans un mode de réalisation préféré, d'un système de diagnostic individuel et personnalisé.

### 1. Description d'un premier mode de réalisation communiquant via un téléphone intelligent.

Le premier mode de réalisation vise spécifiquement un défibrillateur autonome doté de moyens de communication permettant de tirer avantage des fonctionnalités multimédias, notamment mais également de communication mobiles (GSM, GPRS, 3G et des moyens de localisation GPS) d'un téléphone mobile intelligent, et/ou terminaux mobiles.

D'une manière générale, l'architecture interne d'un dispositif défibrillateur et la structure de ses circuits internes sont bien connues d'un homme du métier et il n'est pas nécessaire d'alourdir l'exposé par une description exhaustive de ces circuits.

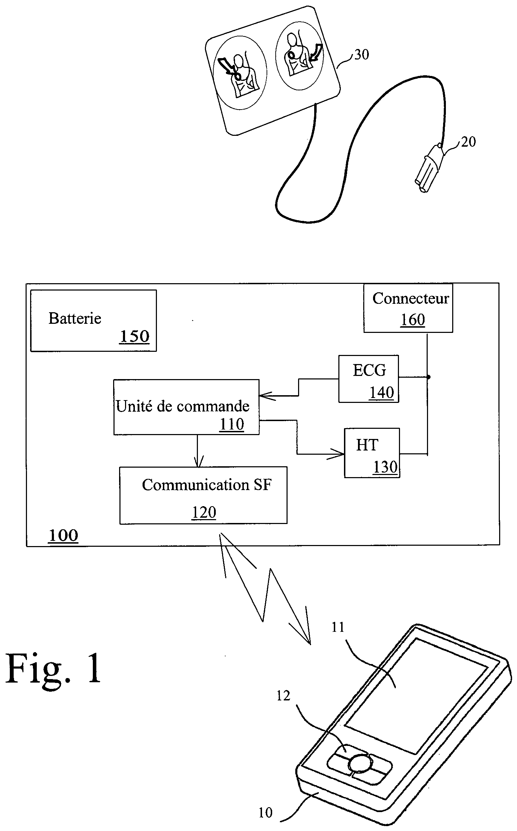

Comme cela est illustré dans la figure 1, l'on considère un défibrillateur, par exemple automatisé externe (DAE) 100 se présentant sous la forme d'un boîtier comportant un connecteur 160 destiné à recevoir un connecteur correspondant 20 relié à une paire d'électrodes 30. Le défibrillateur 100 comporte un bloc fonctionnel 140 destiné à l'analyse et au traitement du signal ECG (Electrocardiogramme) capté par les électrodes 30 ainsi qu'un bloc fonctionnel 130 apte à délivrer un choc électrique Haute-Tension (HT) de plusieurs centaines de volts et plusieurs centaines de Joules.

Le défibrillateur comporte en outre une unité de commande 110 commandant les blocs fonctionnels 130 et 140 ainsi qu'une unité de communication sans fil 120 permettant la communication sans fil avec un dispositif de traitement de données externe 10, comme le téléphone mobile représenté dans la figure. Dans un mode de réalisation particulier, la communication entre le boîtier 100 et le téléphone mobile 10 pourra être basé sur le protocole WIFI et notamment les divers protocoles de communication sans fil régis par les normes du groupe IEEE 802.11 (ISO/CEI 8802-11), mais aussi *Bluetooth,* etc... A titre d'exemple, le téléphone intelligent pourra être un téléphone fonctionnant sous un système d'exploitation open source utilisant le noyau Linux, de type Android. Il est clair cependant qu'un homme du métier pourra adapter a présente divulgation à tout autre système d'exploitation, voire tout autre type de téléphone mobile, smarphones, tablettes tactiles, PDA et terminaux mobiles etc...

Comme cela est connu d'un homme du métier, le bloc fonctionnel ECG 140 sert à capter le signal des électrodes 30 en vue d'une analyse automatique de l'activité électrique du myocarde d'une personne victime d'un arrêt circulatoire, afin de déceler une fibrillation ventriculaire ou certaines tachycardies ventriculaires. Lorsque l'analyse s'avère être positive, l'unité de commande 110 peut activer le chargement de l'appareil - et notamment d'un ensemble de condensateurs internes disposés dans le bloc HT 130, et délivrer les chocs électriques externes transthoraciques, d'intensité appropriée, dans le but de rétablir une activité circulatoire normale.

Le défibrillateur 100 est alimenté par une batterie autonome 150 assurant l'alimentation électrique de tous les circuits électroniques du défibrillateur et notamment la fourniture de l'énergie nécessaire à la délivrance des chocs électriques diffusés par la paire d'électrodes 30.

Dans un mode de réalisation particulier, le boîtier 100 comportera un compartiment destiné à recevoir la paire d'électrodes 30 ainsi que son connecteur 20. Plus spécifiquement, le boîtier pourra adopter la forme particulière d'un pack batterie destiné à un ordinateur portable de manière à permettre un fonctionnement dual, à savoir celui d'une batterie pour cet ordinateur portable, mais également de défibrillateur susceptible de communiquant avec un téléphone mobile dit intelligent.

L'on assure ainsi que dès lors qu'un utilisateur emporte avec lui un ordinateur portable, comportant sa batterie, il emporte également un défibrillateur prêt à être utilisé avec tout téléphone mobile. Ce mode de réalisation spécifique présente en outre l'intérêt de permettre au boîtier 100 de bénéficier des circuits de charge de l'ordinateur portable lui correspondant.

Le dispositif 100 illustré dans la figure 1, et l'unité de commande 110 qu'il comporte sont adaptés à une communication avec le téléphone portable 10 suivant un mode lui permettant de bénéficier des fonctionnalités multimédia - en particulier audio et vidéo - permettant à tout premier secours - même lorsqu'il ne s'agit pas d'un professionnel de la santé - de bénéficier d'un assistant multimédia de haut niveau qui s'avère particulièrement préciser pour l'aider dans la mise en oeuvre du défibrillateur.

A cet égard, lors de l'activation du défibrillateur 100, les fonctionnalités du téléphone mobile 10, notamment l'écran (tactile ou non) 11, l'écran 12 et les fonctions audio, seront toutes réservées au fonctionnement du défibrillateurs de manière à assurer, avec une sécurité maximale le fonctionnement du défibrillateur et la délivrance des chocs électriques.

En particulier, un logiciel d'application ou un jeu de micro instructions mises en oeuvre dans le microprocesseur du téléphone mobile 10 assureront la coopération de ce dernier dans les trois phases majeures du fonctionnement du défibrillateur 100:
phase 1: la mise en fonctionnement du défibrillateur, l'activation de ses circuits internes et la mise en place de la paire d'électrodes 20;
phase 2: l'analyse et le traitement du signal ECG perçu par la paire d'électrodes 30 en vue d'identifier une situation de fibrillation ventriculaire; et
phase 3: la délivrance d'un choc électrique lorsque l'analyse de la phase 2 s'avère positive.

Pendant toute la durée de ces trois phases, l'opérateur bénéficie ainsi d'instructions audibles et visuelles particulièrement claires qui lui sont transmises par le téléphone mobile, le *smartphone*, le PDA etc... communiquant avec le défibrillateur 100, accroissant ainsi l'efficacité et la sécurité de son intervention.

D'une manière générale, l'installation du logiciel d'application ou microprogramme au sein du système d'exploitation du téléphone 10 pourra s'effectuer de manière bien connue, notamment à la suite d'une phase de téléchargement de ce même logiciel depuis une zone de stockage située dans défibrillateur 100, par exemple via les moyens de communication sans fil 120. Dans cette hypothèse, il est à noter que la phase d'installation aura été effectuée préalablement à la mise en oeuvre du défibrillateur, ce qui exclut évidemment une situation d'urgence.

On décrira dans la suite, des modes de réalisation où l'on pourra notamment assurer la mise en oeuvre du défibrillateur même dans une situation d'urgence.

De manière générale, on pourra prévoir également que le logiciel ou le microprogramme installé au sein du téléphone 10 pourra servir également à des opérations de maintenance et de contrôle du défibrillateur, notamment du contrôle du niveau de charge de la batterie interne 150.

Dans un mode de réalisation particulier, le logiciel d'application du défibrillateur provoquera un séquencement de phases d'éveil du défibrillateur pour vérifier de manière périodique l'état de charge de la batterie ainsi que les systèmes électroniques critiques de ce dernier.

### 2. Description d'un second mode de réalisation communiquant avec une tablette tactile

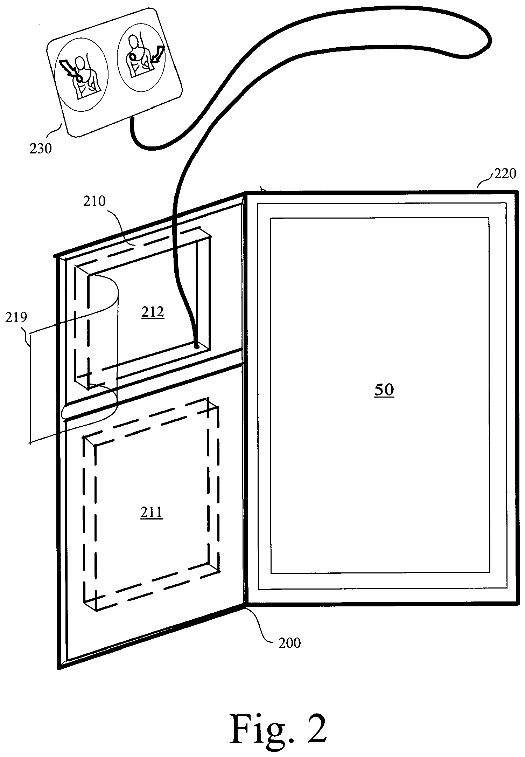

On décrit à présent en relation avec la figure 2 un second mode de réalisation plus adapté à la coopération avec un système de traitement d'informations mobile de type "tablette tactile". On comprendra dans cette acception tous les dispositifs se présentant sous la forme d'un panneau d'affichage et non dotés d'un clavier mécanique comme ceux qui équipent les ordinateurs portables.

La figure 2 illustre une tablette tactile 50 comportant une housse ou coque se présentant sous la forme d'une couverture 200 qui pourra être en tout matériau plastique, cuir, flexible ou non etc...

De préférence, la couverture 200 se présente, à l'instar de celle d'un livre, avec un volet gauche (ou recto) et un volet droit (verso), ce dernier comportant des moyens de fixation de la tablette tactile 210.

Ainsi, l'ensemble couverture-tablette forme l'équivalent d'un *livre* protégé par la couverture 200 comportant les deux volets recto 210 et verso 220.

Suivant un mode de réalisation, l'élément recto 210 présente une certaine épaisseur, par exemple inférieure à 1 cm, et comporte au moins un compartiment interne - comme le compartiment inférieur 211 de la figure 2, destiné à recevoir les circuits électroniques d'un défibrillateur 200 semblable au défibrillateur 100 qui avait été décrit en relation avec la figure 1.

Les progrès de la miniaturisation, notamment l'emploi de condensateurs au tantale permettent d'envisager la mise en place des blocs fonctionnels 210, 230, 240 (équivalents aux blocs 110, 130 et 140 de la figure 1), ainsi qu'un bloc de batterie lon-lithium permettant l'alimentation électrique du défibrillateur.

De préférence, le volet gauche 211 comporte en outre, sur sa partie supérieure, un second compartiment 212 destiné à recevoir une paire d'électrodes 230 semblables aux électrodes 30 illustrées dans la figure 1.

De préférence, les électrodes 230 ne sont accessibles depuis le compartiment 212 qu'au moyen d'un accès scellé assurant un usage unique du défibrillateur de manière à accroître la sécurité de fonctionnement, comme ce que l'on a représenté dans la figure 2 par la déchirure d'une pellicule 219, qui empêchait jusqu'alors l'accès au compartiment 212 et qui est rabattue à gauche du volet recto 210.

Grâce à la mise en place de ce scellé 219, l'on assure l'intégrité du défibrillateur pour une unique utilisation en toute sécurité, et l'on prévient en particulier une décharge inopportune de la batterie contenue dans le compartiment 211 du volet gauche (recto).

De la même manière que précédemment, préalablement à l'utilisation du défibrillateur contenu dans le compartiment 211, l'on vient installer par des moyens appropriés un logiciel d'application dans le système d'exploitation de la tablette tactile 50 de manière à permettre notamment la communication sans fil avec le défibrillateur mais également diverses fonctions de maintenance et de contrôle, notamment de contrôle épisodique régulier (tous les mois par exemple) du niveau de charge de la batterie interne stockée dans le compartiment 211. Dans un mode de réalisation particulier, le logiciel installé procède à la vérification de l'intégrité du scellé 219, par tout système quelconque, tel que par exemple un fil de rupture, un détecteur capacitif ou de lumière etc....

Grâce au concours du défibrillateur disposé dans le compartiment 211 et des fonctionnalités multimédias mises à disposition par la tablette tactile, l'utilisateur premier secours tirer avantage des fonctionnalités audio-vidéo et bénéficier d'un tutoriel ou d'une assistance précieuse lors de la mise en oeuvre du défibrillateur.

De préférence, le logiciel mis en oeuvre dans la tablette tactile détecte la rupture du scellé 219 et met en oeuvre les fonctionnalités de géo-positionnement de manière à transmettre un message d'alerte à une équipe de secours permettant ainsi à l'opérateur premier secours de se concentrer sur la mise en oeuvre du défibrillateur, tout en sachant qu'une équipe professionnelle est appelée à se rendre immédiatement sur les lieux.

A cet égard, le logiciel pourra utiliser les fonctionnalités de télécommunications avancées présentes dans la tablette tactile, notamment les accès GSM, GPRS, 3G et 4G potentiellement présentes dans le système.

Ainsi, en même temps que l'utilisateur met en oeuvre le défibrillateur situé dans le compartiment 211, un message d'alerte, généré automatiquement dès la rupture du scellé 219 est transmis à un centre d'appel adéquat, accroissant ainsi l'efficacité du système de secours.

Dans un mode de réalisation particulier, le volet gauche 210 comporte en outre un connecteur USB ou micro-USB (Universal Serial Bus) permettant la connexion à la tablette tactile ou à l'assistant digital portable (PDA) et pouvant servir, soit à l'installation d'un logiciel adéquat au sein de cette dernière, soit la recharge ou le maintien en charge de la batterie autonome stockée dans le compartiment 211.

Dans cette dernière configuration, l'on peut également prévoir que la batterie présente dans le compartiment 211 puisse également servir de batterie de secours pour la tablette tactile 50 grâce à son port USB. Dans ce cas, le logiciel ou microprogramme installé dans le système d'exploitation de cette batterie assurera le suivi de l'état de charge et de décharge de cette batterie et générera les alertes à l'attention de l'utilisation pour l'inviter à recharger dès que possible la batterie du défibrillateur. Dans cette configuration, l'on pourra avantageusement prévoir des circuits électroniques permettant de tracer les phénomènes de charge/décharge de la batterie pour permettre de suivre précisément les conditions de fonctionnement et d'utilisation de cette dernière. Les informations adéquates pourront être notamment transmises au fabricant du défibrillateur par le logiciel d'application s'exécutant sur la tablette tactile au moyen des outils de communication mobiles (WIFI, GSM, GPRS, 3G, 4G etc..)

L'on voit sur les exemples qui sont décrits à quel point peut être fertile et avantageuse la combinaison des circuits du défibrillateur avec un téléphone mobile et/ou une tablette tactile.

Mais comme on va le voir à présent avec le troisième mode de réalisation, c'est avec un ordinateur portable, que la coopération des systèmes apparaît la plus féconde.

### 3. Description d'un troisième mode de réalisation communiquant avec un ordinateur portable

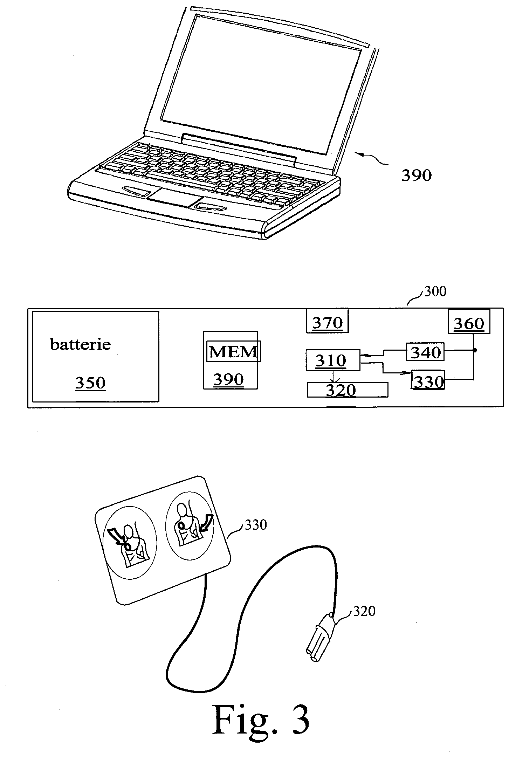

Dans le troisième mode de réalisation qui est décrit à présent en référence avec la figure 3 le défibrillateur est d'emblée installé dans un pack de batterie destiné à correspondre à un modèle spécifique d'un ordinateur portable 390 d'une marque connue.

L'on pourra ainsi prévoir de couvrir, avec des circuits électroniques communs, une large gamme d'ordinateurs portables.

En se référant à la figure 3, l'on voit que le pack batterie/défibrillateur 300 comporte, outre une batterie autonome 350, un connecteur 360 destiné à permettre le branchement d'un connecteur 320 d'une paire d'électrodes 330.

Dans un mode de réalisation particulier, le boîtier du pack batterie/défibrillateur 300 comporte un compartiment permettant de loger la paire d'électrodes 330 et son connecteur 320.

Le pack batterie/défibrillateur 300 comporte en outre un bloc fonctionnel 340 destiné à l'analyse et au traitement du signal ECG ainsi qu'un bloc fonctionnel 330 apte à délivrer le choc électrique HT. Il comporte également une unité de commande 310 commandant les blocs fonctionnels 330 et 340.

Optionnellement, le block batterie/défibrillateur comporte également une unité de communication sans fil 320 permettant une éventuelle communication sans fil, soit avec l'ordinateur portable, soit avec tout autre système de traitement de données.

D'une manière générale, l'ordinateur portable 390 comporte un système d'exploitation quelconque, tel que notamment Windows XP ou Windows 7 de l'éditeur MICROSOFT Corp. Alternativement, l'on pourra envisager un système d'exploitation de type LINUX.

Dans un mode de réalisation particulier, le bloc de commande 310 communique avec le système d'exploitation de l'ordinateur portable de manière à permettre au défibrillateur de bénéficier des fonctionnalités multimédias de ce dernier.

Dans un mode de réalisation préféré, pour éviter tout risque afférent au système d'exploitation s'exécutant dans l'ordinateur portable, l'on prévoit au sein du défibrillateur 300 une zone mémoire 390 comportant un microprogramme de démarrage, écrit en un langage quelconque, permettant de prendre en charge toute la procédure de démarrage de l'ordinateur portable grâce à un connecteur USB connecté à un port USB 370.

De cette manière, l'on court-circuite l'exécution du système d'exploitation pour assurer que la totalité des ressources de l'ordinateur portable sont bien effectivement affectées au fonctionnement du seul défibrillateur 300.

A cet effet, dans un mode de réalisation particulier, nullement limitatif, le pack batterie/défibrillateur 300 de la figure 3 comporte des circuits de commutation spécifiques pour permettre une commutation quasi immédiate sur le logiciel du défibrillateur .

A cet égard, le dispositif 300 comporte des circuits de commutation détectant la connexion de la paire d'électrodes 330 sur le connecteur 360, dans le but de provoquer une rupture d'alimentation de l'ordinateur portable 400. Créant de ce fait, d'une manière sans doute brutale mais particulièrement efficace, l'extinction du système d'exploitation qui est en cours d'exécution.

En outre, le pack batterie/défibrillateur comporte des systèmes de commutation supplémentaires (intégrés dans le module de commande 310) pour n'autoriser l'alimentation électrique de l'ordinateur qu'à condition que le port USB 370 n'est branché sur un des ports USB de l'ordinateur portable.

Cette disposition, permet d'assurer un redémarrage de la machine, hors système d'exploitation, sur un microprogramme stocké dans la mémoire 390 localisée dans le pack batterie/défibrillateur 300, sous réserve de la configuration adéquate préalable de la séquence de "*boot*" du BIOS (*Basic Input Output Sequence*) de la carte mère de l'ordinateur portable.

De ce fait, l'on parvient avantageusement à éviter que ne se mettent en oeuvre les procédures de test du système d'exploitation à la suite de l'arrêt brutal de ce dernier, et qui sont clairement incompatibles avec le démarrage du défibrillateur 300.

On parvient ainsi à éviter au premier secours la perte d'un temps crucial.

De préférence le microprogramme est un logiciel développé en Linux, ou voire même en DOS (*Disk Operating System*) permettant un démarrage ultra-rapide.

Alternativement, l'on pourra recourir à tout autre langage , tel que le C++, lequel peut servir au fonctionnement d'un ordinateur dénué de tout système d'exploitation. L'homme du métier se reportera notamment aux derniers développement en matière de programmation hors système d'exploitation (désigné dans la littérature anglo-saxonne par l'appellation "*bare computing*"). Voir notamment la référence:
*"*How to run C++ applications on a bare PC?" de Dr Ramesh K. Karne et al,.

L'on assure ainsi un démarrage quasi immédiat de l'ordinateur portable, avec l'assurance que toutes les ressources de l'ordinateur seront réservées au seul défibrillateur 300. Le premier secours est alors assuré de ce qu'il pourra bénéficier des ressources multimédias, notamment audio-vidéos mais également des fonctionnalité de géolocalisation et de communications mobiles lui permettant de bénéficier alors d'un tutoriel, d'une assistance efficace et précieuse pour assurer un fonctionnement approprié du défibrillateur 300 .

### 4. Description d'un quatrième mode de réalisation

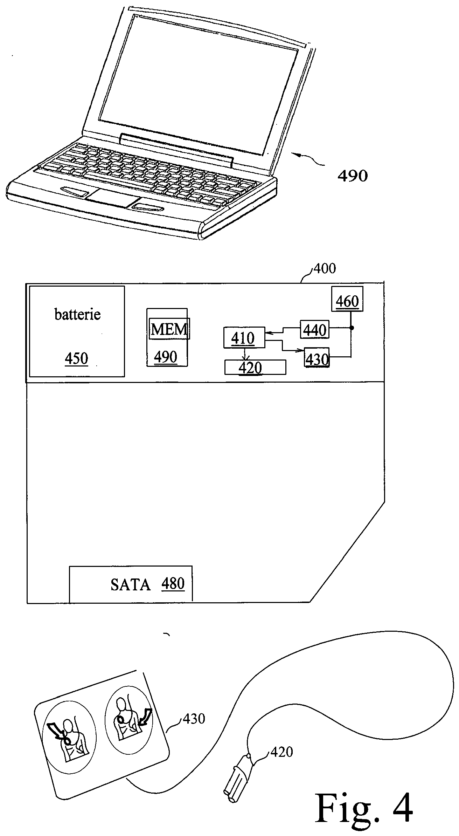

Dans le quatrième mode de réalisation qui est décrit à présent en relation avec la figure 4, l'on voit que le défibrillateur se présente sous la forme d'un "caddie" 400 destiné à occuper la place d'un lecteur de CDR/DVD d'un ordinateur portable. En particulier, le "*caddie*" comporte un port SATA 480 destiné à venir se connecter sur le port correspondant de la carte mère de l'ordinateur portable.

D'une manière similaire au pack batterie/défibrillateur de la figure 3, le défibrillateur 400 comporte une batterie autonome 450 (qui pourra être de capacité plus réduite que la batterie 350 de la figure 3 puisqu'elle n'est pas destinée à servir à l'alimentation électrique de l'ordinateur portable), et un connecteur 460 destiné à permettre le branchement d'un connecteur 420 d'une paire d'électrodes 430. De préférence, la paire d'électrodes 430 pourra se loger spécifiquement dans un compartiment spécifique du "caddie" 400.

Le défibrillateur 400 comporte en outre, de manière similaire au défibrillateur 300 de la figure 3, un bloc fonctionnel 440 destiné à l'analyse et au traitement du signal ECG ainsi qu'un bloc fonctionnel 430 apte à délivrer le choc électrique HT. Il comporte également une unité de commande 410 commandant les blocs fonctionnels 430 et 440. Le défibrillateur 400 comporte également une zone mémoire 490 destiné à recevoir les micro instructions servant au démarrage de l'ordinateur portable, lequel pourra être effectué cette fois ci directement depuis le connecteur SATA 480 enfiché sur le bus correspondant de la carte mère.

Comme précédemment, optionnellement, le block batterie/défibrillateur comporte également une unité de communication sans fil 420 permettant une éventuelle communication sans fil, soit avec l'ordinateur portable, soit avec tout autre système de traitement de données.

D'une manière générale, l'ordinateur portable 490 comporte un système d'exploitation quelconque, tel que notamment Windows XP ou Windows 7 de l'éditeur MICROSOFT Corp. Alternativement, l'on pourra envisager un système d'exploitation de type LINUX ou tout autre système d'exploitation.

On pourra également prévoir, comme pour le troisième mode de réalisation, des circuits de commutations (intégrés dans le bloc 410) permettant de rendre actif le défibrillateur 400 dès lors que l'opérateur aura branché la paire d'électrodes. En particulier, les circuits de commutation pourront ne rendre actif la connexion via la port SATA 480 uniquement lorsque la connexion de la paire d'électrodes aura été effectué via le connecteur 460.

Pour assurer une disponibilité immédiate des ressources de l'ordinateur portable au seul défibrillateur 400, un mécanisme spécifique est prévu basé sur la détection du branchement des électrodes 430, de manière à sécuriser le fonctionnement de l'ordinateur lors de la mise en oeuvre du défibrillateur et éviter toute interruption dans le fonctionnement de ce dernier.

Une fois les ressources de l'ordinateur portable alloué au défibrillateur 400, l'opérateur premier secours dispose alors des fonctionnalités multimédias présentes dans l'ordinateur portable, lui assurant ainsi le support d'un assistant efficace lors de l'opération de défibrillation.

Comme on le voit, la présente divulgation permet une multitude de configurations permettant d'accroître la simplicité, l'efficacité ainsi que la sécurité du premier secours.

L'on notera en particulier que les modes de réalisation qui sont décrits ne sont nullement exclusifs les uns par rapport aux autres. En effet, comme on va le voir, le troisième mode de réalisation qui vient d'être décrit, outre qu'il permet le fonctionnement d'un ordinateur portable (en lui assurant son alimentation électrique) et également avec un ordinateur portable en faisant bénéficier le premier secours des fonctionnalités multimédias de l'ordinateur portable, on pourra également avantageusement prévoir que ce "pack batterie" qui vient d'être décrit puisse également fonctionner, grâce au bloc de communication 320, avec un téléphone mobile intelligent, ou une tablette tactile comme cela a été décrit en relation avec la figure 1.

L'on constate ainsi la grande possibilité d'adaptation permettant, dans bien des situations d'urgence, d'avoir une aide effective et critique au premier secours d'une victime d'un accident cardio-respiratoire.

### 5. Description d'un cinquième mode de réalisation

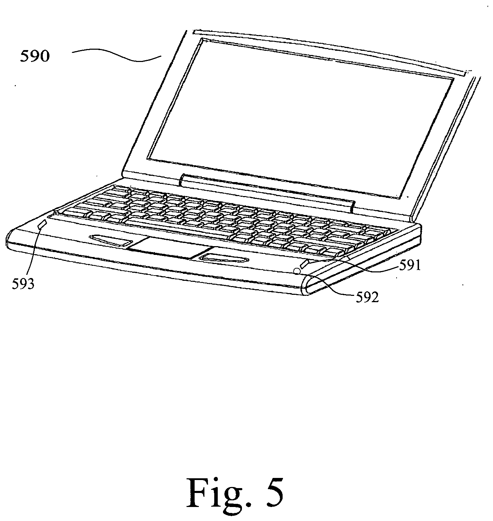

L'on décrit à présent, en relation avec la figure 5 un cinquième mode de réalisation ses déclinant sous la forme d'un dispositif de traitement mobile de l'information (désigné *Information Handling System* dans la littérature anglo-saxonne) - par exemple un ordinateur portable 590 ou une tablette doté d'un clavier mobile - comportant, de fabrication, une unité complète de défibrillation.

De cette manière, un constructeur informatique peut être amené à concevoir et réaliser, au coût le plus avantageux, un dispositif complet comportant tous les circuits électroniques permettant d'assurer un traitement thérapeutique de défibrillation là où cela s'avère nécessaire. Le dispositif 590 sera ainsi doté d'un connecteur permettant la connexion des électrodes qui pourront être logées dans tout réceptacle quelconque du dispositif ou d'un accessoire associé comme sa coque ou sac de transport. Dans le cas d'une tablette l'on pourra clairement utiliser la coque spécifique décrite en relation avec la figure 2. Par ailleurs, le dispositif de défibrillation étant partie intégrante du dispositif 590, ce même système pourra ainsi être doté du logiciel adéquat pour permettre l'utilisation du défibrillateur par le grand public, et notamment la fonctionnalité de tutoriel et les fonctionnalités de mise en service des circuits électroniques pour permettre la délivrance du choc thérapeutique salvateur, , tel que cela a été décrit précédemment en relation avec les modes de réalisations 1 à 4.

Dans un mode de réalisation particulier, le dispositif de traitement de données 590 est adapté de manière à assurer, outre la fonction ***thérapeutique*** décrite précédemment, une fonction supplémentaire particulièrement avantageuse de **diagnostic médical** que l'on va décrire à présent plus en détail pour illustrer la grande flexibilité de ce mode de réalisation.

L'on dispose au sein de l'ordinateur portable/tablette une centrale de diagnostique comportant au moins un capteur permettant de suivre une donnée physiologique de l'utilisateur au fur et à mesure que ce dernier se sert du dispositif pour ses activités quotidiennes, et notamment le pouls, la tension, la température, la pression de saturation en oxygène, P.H CT° etc....

En particulier, on dispose d'un capteur infrarouge ou photocapteur 592 (sur la figure 5) permettant de saisir le rythme cardiaque ainsi que la saturation d'oxygène et ce, au moyen d'une analyse de la vitesse de déplacement des globules rouges.

De cette manière, l'utilisateur du système peut disposer, alors qu'il consulter ses courriels ou travaille sur ses dossiers, d'informations pertinentes concernant sa pression, sa fréquence cardiaque...De manière préventive, le logiciel de diagnostique présent dans le système peut vérifier au jour le jour que les données recueillies restent confinées dans des valeurs acceptables et que cet utilisateur ne fait pas l'objet, par exemple, d'extrasystoles.

Dans un autre mode de réalisation, le photo capteur servant à cette analyse est combiné avantageusement avec un capteur qui est conventionnellement présent sur le système, à savoir un capteur d'empreinte digitale 591. De préférence, l'on dispose le capteur infrarouge 592 à proximité du capteur d'empreinte digitale 591 de manière à permettre, en une seule opération, la saisie de l'empreinte digitale - nécessaire au démarrage du système - mais également la saisie de la coure de pression et de fréquence cardiaque ainsi que la mesure de la saturation d'oxygène.

Ainsi, dans la continuité d'un « même geste » , l'utilisateur peut satisfaire aux conditions de sécurité permettant le démarrage du système tout en obtenant des données médicales d'un grand intérêt pour lui.

De cette manière l'on obtient une combinaison particulièrement avantageuse des fonctions de « sécurité » propres au système 590 et de fonctions de diagnostic médicales toutes aussi utiles pour l'utilisateur de ce même système.

Dans un autre mode de réalisation, l'on dispose en outre sur le dispositif 590 un jeu de deux électrodes bipolaires, respectivement gauche et droite, permettant une saisie de potentiel dans une configuration bipolaire. Une telle disposition permet alors au dispositif 590 et au logiciel de diagnostic médical en particulier de réaliser, au démarrage de la machine ou à tout moment opportun, une saisie de données pour la génération d'un électro-cardiogramme.

De préférence, l'une des électrodes (droite par exemple) est disposée à proximité du capteur infrarouge ou même, comme cela est illustré dans la figure 5, est commune avec le capteur d'empreinte digitale 591 de manière à permettre la saisie au moyen du même doigt (droit) servant à la prise d'empreinte digitale et à la prise du rythme cardiaque, tandis que l'autre électrode (gauche) 593 se situe dans un emplacement à gauche du dispositif.

De manière générale, le logiciel de diagnostic procède à l'enregistrement périodique de données médicales de l'utilisateur du système, au sein d'un espace mémoire protégé, par exemple dans une clé USB médicale .

Ces données pourront servir en combinaison d'informations médicales générales saisies par l'utilisateur et susceptibles de constituer des facteurs de risque (age, poids, antécédents familiaux, fumeur ou non etc...) en réponse à un questionnaire généré automatiquement par le logiciel de diagnostic et auquel répond l'utilisateur.

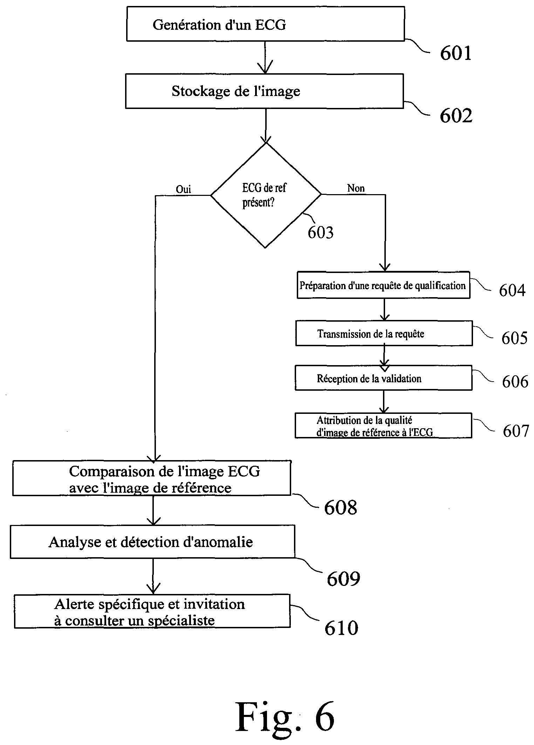

Dans un mode de réalisation particulier, outre le stockage des ECG générés périodiquement, le logiciel de diagnostic procède à une vérification périodique de l'électrocardiagramme généré automatiquement grâce aux deux électrodes bipolaires. A cet effet, une image de référence est générée par le logiciel, notamment lors de la première utilisation du dispositif par l'utilisateur, et qualifiée comme telle au moyen d'une procédure spécifique que l'on décrit à présent en relation avec la figure 6.

Le procédé de la figure 6 démarre avec une **étape 601** au cours de laquelle, lors du démarrage de l'ordinateur portable/tablette, une image ECG est générée au moyen des deux électrodes bipolaires présentes sur le système, comme cela a été décrit précédemment. D'une manière générale, le traitement analogique (amplification/filtrage) et numérique (conversion analogique/numérique) permettant la génération d'une telle image est bien connue d'un homme du métier et ne sera pas décrite plus avant.

Puis, dans une **étape 602,** le procédé procède au stockage de cette image ECG. Dans un mode de réalisation particulier, cette image ECG est stockée dans une zone spécifique de la mémoire du système, voire même une clé USB à vocation médicale, de manière à constituer une base de données particulièrement nourrie et utile pour le propriétaire du dispositif.

Puis le procédé détermine, au moyen d'un test dans une **étape 603,** si une image, qualifiée comme image de référence est déjà présente dans le système.

Si une telle image de référence est présente dans le système, le procédé poursuit directement avec une étape 608.

Dans le cas contraire, en l'absence d'une image de référence, le procédé poursuit avec **la préparation d'une requête de qualification** au cours d'une **étape 604.** A cet effet, le procédé procède à la préparation d'une requête intégrant l'image ECG saisie avec des informations supplémentaires, par exemple administrative (nom de l'utilisateur) voire mêmes médicales. Optionnellement, cette étape de préparation pourra suivre une phase préliminaire de validation « interne » de l'image ECG par le logiciel de diagnostique de manière à s'assurer que l'image ECG obtenue lors de l'étape 601 présente un niveau de qualité suffisant pour pouvoir être utilisée dans la phase de préparation de l'étape 604.

Dans un mode de réalisation particulier, afin de renforcer la sécurité et la pertinence du diagnostic qui sera ultérieurement effectué par le logiciel de diagnostic, l'image ECG qui est soumise à des fins de validation est signée au moyen de données d'identification appartenant à l'utilisateur.

L'on pourra par exemple avantageusement associer l'image ECG capturée lors de l'étape 601, alors que l'utilisateur avant posé deux doigts sur les électrodes bipolaires, avec une image prise par la caméra (*webcam*) au moment de la capture de l'ECG ou, alternativement (voire cumulativement), avec l'empreinte digitale saisie simultanément ou conjointement au moyen du capteur d'empreinte digitale.

Ainsi, l'on parvient à associer irrémédiablement une image ECG - qui pourra être ultérieurement qualifiée comme image de référence, avec une donnée d'identification (empreinte digitale) particulièrement sûre et qui permettra au cardiologue ou à l'expert validant la qualification de conserver en même temps l'image ECG qu'il aura validé avec l'empreinte digitale du doigt qui aura servi à la génération de cette image. La sécurité de cette association entre l'image ECG et l'empreinte digitale pourra être renforcée d'avantage par divers moyens de signatures, de hashage bien connus d'un homme du métier.

D'une manière pratique, une fois la requête préparée lors de l'étape 604, celle-ci pourra aboutir à différents formats, comme par exemple un fichier XML intégrant l'image JPG de l'électrocardiogramme, voire même un message électronique. Le fichier XML pourra d'ailleurs intégrer toute autre donnée médicale susceptible d'être collectée par le dispositif 590.

Puis, dans une **étape 605,** le procédé mis en oeuvre par le logiciel de diagnostic procède à l'envoi de la requête de qualification vers un serveur distant spécialisé (non illustré dans la figure 6).

D'une manière générale, la transmission de la requête de qualification pourra utiliser tous les moyens modernes de télétransmission, filiaires, sans fils, et s'appuyant notamment sur les protocoles de transmission (IP) en usage dans le réseau Internet.

Par ailleurs, le serveur distant est un serveur spécialisé, géré par des experts compétents dans le domaine de la cardiologie et pouvant valider l'image ECG reçue lors de l'étape 605.

En pratique, l'on pourra également prévoir que le serveur distant sera un système informatique détenu par le cardiologue habituel de l'utilisateur du système , lequel pourra alors examiner à loisir l'image ECG générée par le dispositif 590 et valider la qualification d'image de référence. Dans l'hypothèse où l'image ECG est signée par des données d'identification de l'utilisateur, la validation par le cardiologue traitant permettant à ce dernier de s'assurer que l'image de référence ne pourra êtte détournée et utilisée par un autre système de diagnostic que celui qui apparaît à son utilisateur.

Cette validation de la qualification est alors reçue au cours d'une **étape 606** par le dispositif 590 qui peut alors, dans une **étape 607,** stocker définitivement l'image ECG avec la qualification d'image de référence. Cette image pourra alors servir à des fins de comparaison, notamment lors d'une analyse des images ECG qui seront générées quotidiennement lors de l'utilisation du dispositif 590.

En effet, à la suite du test de l'étape 603, si le procédé a conclu en la présence d'une image de référence stockée au sein du système, celui-ci procède ensuite, dans une **étape 608,** à une comparaison de l'image ECG nouvellement acquise avec cette image de référence.

Cette comparaison se poursuit ensuite, lors d'une **étape 609,** par une analyse circonstanciée de l'image ECG nouvellement acquise de manière à permettre, le cas échéant, la détection d'anomalie particulières, mises en évidence lors de la comparaison de l'étape 608. Dans un mode de réalisation particulier, le procédé procède au monitoring des systoles, des arythmie et toute anomalie susceptible d'être détectée sur l'ECG.

Si une telle anomalie est détectée, alors le procédé poursuit, lors d'une étape 610, par la génération d'une alerte spécifique destinée à l'utilisateur du dispositif 590, voire même, le cas échéant, au serveur distant.

Le système qui vient d'être décrit présente un grand intérêt d'un point de vue médical pour l'utilisateur de l'ordinateur portable ou de la tablette 590. En effet, sans que celui-ci s'en préoccupe vraiment, il lui est offert, grâce aux dispositifs et procédures décrits précédemment, la possibilité de générer automatiquement une image ECG de référence mais également des images périodiques qui pourront être utilement comparées à l'image de référence dans le but de détecter diverses anomalies et situations de risque cardiaque. Ainsi, six mois après avoir acquis le système et généré sa première image ECG (supposée normale), le logiciel de diagnostic pourra être en mesure de l'alerter sur l'existence de divers problèmes apparaissant sur l'ECG, par exemple, une anomalie de la repolarisation, une anomalie de la contraction auriculaire etc...

Ainsi prévenu et alerté , l'utilisateur pourra consulter rapidement.

En particulier, certaines situations particulièrement préoccupantes pourront être prévenues, notamment la situation d'infarctus silencieux qui, comme cela est connu, ne se manifestent pas par les symptômes habituellement rencontrés (mal dans le bras, mal de mâchoire, étau dans la poitrine). Le patient susceptible d'en souffrir ne peut ainsi aisément détecter le risque (autrement qu'en allant consulter régulièrement son cardiologue). En revanche, la situation d'infarctus silencieux se détecte de manière électrique grâce à une analyse de l'ECG, comme l'analyse faite lors de l'étape 609 de la figure 6. Ainsi, sous réserve qu'il utilise suffisamment fréquemment son système (notamment pour accéder à ses courriels), l'utilisateur peut être prévenu assez tôt qu'il présente un risque important ou, même, qu'il a fait un infarctus silencieux ... Face à un tel risque, il peut alors aller consulter rapidement. Cet exemple montre ainsi le grand intérêt de la présente divulgation.

Dans des modes de réalisation particuliers, le dispositif 590 pourra être enrichi de plusieurs fonctionnalités de diagnostique supplémentaires qui pourront utilement venir compléter l'image ECG :
- la prise de pression artérielle au moyen d'un brassard adapté, par exemple pour être connecté par un port USB sur le dispositif 590 ;
- des données relatives au monitoring de l'apnée du sommeil . A cet effet, le dispositif 590 comportera un port permettant la connexion de deux électrodes supplémentaires placés sur le thorax ou, alternativement, permettra une connexion par des moyens sans fil à un dispositif autonome auquel seront connectées ces deux électrodes. D'une manière générale, la prise de données caractéristiques de monitoring de l'apnée du sommeil est bien connu d'un homme du métier et il n'est pas besoin d'alourdir l'exposé avec des détails supplémentaires.
- Le contrôle de la glycémie au moyen d'un appareil adapté à cet effet et susceptible d'être connecté par des moyens de communications (port USB ou moyen sans fil) au dispositif 590.
- Le contrôle de la courbe de poids au moyen d'une balance adéquate susceptible de communiquer avec le logiciel de diagnostique.

L'invention est définie dans les revendications qui suivent. Les exemples, aspects ou modes de réalisation divulgués dans la présente description qui ne sont pas couverts par lesdites revendications, ne sont fournis qu' à titre indicatif seulement, et ne font pas partie de la présente invention.

## Revendications

1. Dispositif mobile de traitement de données sous la forme d'un ordinateur portable (390, 490, 590), une tablette tactile ou un téléphone intelligent (10), ledit dispositif mobile étant configuré pour mettre en oeuvre une procédure de démarrage sécurisée par la saisie d'une empreinte digitale et comportant en outre de fabrication :
un circuit électronique de défibrillation (100); une batterie (150) destinée à alimenter ledit circuit de défibrillation; un logiciel d'assistance à l'utilisation pour permettre l'utilisation du défibrillateur par le grand public;
une centrale de diagnostic et de monitoring médical, comportant au moins un capteur permettant de suivre une donnée physiologique de l'utilisateur du dispositif, ladite donnée physiologique de l'utilisateur consistant en l'un des éléments suivants : le pouls, la tension, la température, la pression de saturation en oxygène, P.H.C.T , ledit dispositif mobile étant configuré pour saisir et stocker ladite donnée physiologique de l'utilisateur conjointement avec la mise en oeuvre de la procédure de démarrage afin de permettre un stockage sécurisé de ladite donnée physiologique..

2. Dispositif mobile de traitement de données selon la revendication 1, comportant un capteur infrarouge destiné à la saisie de la saturation en oxygène et de la fréquence cardiaque de l'utilisateur du dispositif mobile , ainsi que son enregistrement périodique au sein d'une zone de stockage contrôlée par le logiciel de diagnostic

3. Dispositif mobile de traitement de données selon la revendication 2, dans lequel ledit capteur infra-rouge est localisé à proximité immédiate d'un capteur d'empreinte digitale présent à des fins de sécurité, de manière à assurer conjointement :
- la saisie de la courbe de pression et de fréquence cardiaque en même temps que la prise d'empreinte digitale destinée à l'activation du système ;
- la signature des courbes de pression et de fréquence cardiaque par une information dérivée du capteur d'empreinte digitale de manière à associer irréversiblement les données saisies par le capteur infrarouge et l'empreinte digitale de l'utilisateur

4. Dispositif mobile selon la revendication 3 comportant un dispositif de monitoring de l'apnée du sommeil.

5. Dispositif mobile de traitement de données selon la revendication 3 comportant deux électrodes permettant la capture d'un électrocardiogramme dans une configuration bipolaire et :
- des moyens de stockage de l'électrocardiogramme au sein du dispositif;
- des moyens d'analyse de l'électrocardiogramme et de comparaison avec une image de référence ;
- en réponse à ladite comparaison, des moyens d'alerte de l'utilisateur du dispositif

6. Procédé de monitoring pour un dispositif mobile tel que défini dans la revendication 1 à 5, comportant les étapes suivantes:
- la capture d'une image ECG de référence, simultanément à une donnée d'identification de l'utilisateur ;
- la signature de l'image de référence au moyen de la donnée d'identification de l'utilisateur,
- la transmission de l'image de référence à un serveur ou à un système distant en vue d'une validation de l'image de référence ;
- réception d'une confirmation de validation de l'image de référence ;
- stockage de l'image validée comme image de référence et utilisation de cette dernière à des fins de comparaison et d'analyse avec des captures ultérieures d'ECG par ledit système mobile.

7. Procédé de monitoring pour un dispositif mobile tel que défini dans la revendication 6, dans lequel les enregistrements ECG ultérieurs sont stockés sur un support de stockage spécifique à l'utilisateur.

8. Dispositif mobile de traitement de données tel que défini dans l'une des revendication 1 à 5, dans lequel ledit défibrillateur est configuré sous la forme d'un kit destiné à se substituer à une batterie d'origine du fabricant.

9. Dispositif mobile de traitement de données selon la revendication 8 comportant des moyens permettant d'organiser un double fonctionnement avec ledit ordinateur portable ou ultra-portable:
- un premier fonctionnement dans le cadre d'un système d'exploitation natif présent au sein de l'ordinateur, destiné à la mise en oeuvre de procédures non critiques de maintenance du système de défibrillation;
- un second fonctionnement forçant l'extinction du système d'exploitation préexistant au sein de l'ordinateur, de manière à réserver l'intégralité des ressources au dispositif de défibrillation.

10. Dispositif mobile de traitement des données selon la revendication 9 comportant un connecteur de type USB permettant d'être utilisé pour un démarrage à froid de l'ordinateur.

11. Dispositif mobile de traitement des données selon la revendication 8 et 10 comportant:
- des moyens permettant de détecter le branchement des électrodes de défibrillation;
- des moyens de commande, en réponse à la détection dudit branchement, pour:
- forcer l'extinction du système d'exploitation en réponse à la détection du branchement des électrodes de défibrillation;
- activer le connecteur USB de manière à permettre un démarrage à froid (boot) immédiat via ledit connecteur USB.

## Patentansprüche

1. Mobile Datenverarbeitungsvorrichtung in Form eines Laptops (390, 490,590), eines Tablets oder eines Smartphones (10), wobei besagte mobile Vorrichtung für eine gesicherte Startprozedur durch Erfassung eines Fingerabdrucks konfiguriert ist und außerdem folgendes umfasst:
eine elektronische Defibrillationsschaltung (100); eine Batterie (150) zur Versorgung besagter Defibrillationsschaltung; eine Benutzungshilfe-Software, um die Benutzung des Defibrillators durch die Öffentlichkeit; zu gestatten;
eine Diagnose- und Patientenüberwachungszentrale mit mindestens einem Sensor, um ein physiologisches Datum des Nutzers der Vorrichtung zu verfolgen, das aus einem der folgenden Elemente besteht: der Puls, die Spannung, die Temperatur, der Sauerstoffsättigungsdruck, P.H.C.T, wobei besagte mobile Vorrichtung konfiguriert ist, um besagtes physiologische Datum des Nutzers zusammen mit der Durchführung der Startprozedur zu erfassen und zu speichern, um eine gesicherte Speicherung des besagten physiologischen Datums zu gestatten.

2. Mobile Datenverarbeitungsvorrichtung nach Anspruch 1, mit einem Infrarotsensor zur Erfassung der Sauerstoffsättigung und der Herzschlagfrequenz des Nutzers der mobilen Vorrichtung, sowie zu seiner periodischen Registrierung in einem von der Diagnosesoftware kontrollierten Speicherbereich.

3. Mobile Datenverarbeitungsvorrichtung nach Anspruch 2, wobei besagter Infrarotsensor gleich neben einem digitalen Fingerabdrucksensor liegt, der zur Sicherheit dient, um folgendes gemeinsam zu garantieren:
die Erfassung der Druck- und der Herzfrequenzkurve gleichzeitig mit der Aufnahme eines digitalen Fingerabdrucks, der zur Aktivierung des Systems dient;
die Signatur der Druck- und der Herzfrequenzkurve durch eine Information, die vom digitalen Fingerabdrucksensor abgeleitet wird, um die vom Infrarotsensor erfassten Daten und den digitalen Fingerabdruck des Nutzers unumkehrbar zu verbinden.

4. Mobile Vorrichtung nach Anspruch 3, die eine Vorrichtung zur Überwachung der Schlafapnoe umfasst.

5. Mobile Datenverarbeitungsvorrichtung nach Anspruch 3, mit zwei Elektroden zur Erfassung eines Elektrokardiogramms in einer bipolaren Ausgestaltung sowie:
Mitteln zum Speichern des Elektrokardiogramms in der Vorrichtung;
Mitteln zur Analyse des Elektrokardiogramms und zum Vergleich mit einem Bezugsbild;
als Reaktion auf besagten Vergleich, Mitteln zur Alarmierung des Nutzers der Vorrichtung.

6. Überwachungsverfahren für eine mobile Vorrichtung nach Anspruch 1 bis 5, das die folgenden Schritte umfasst:
die Erfassung eines EKG-Bezugsbildes gleichzeitig mit einem Datum zur Identifizierung des Benutzers;
die Signatur des Bezugsbildes mittels des Identifizierungsdatums des Nutzers
die Übertragung des Bezugsbildes an einen Server oder an ein entferntes System in Hinblick auf eine Validierung des Bezugsbildes;
Empfang einer Validierungsbestätigung des Bezugsbildes;
Speicherung des validierten Bildes als Bezugsbild und Benutzung des letzteren zum Vergleich und zur Analyse mit weiteren EKG-Aufnahmen durch besagtes mobiles System.

7. Überwachungsverfahren für eine mobile Vorrichtung nach Anspruch 6, wobei die weiteren EKG-Aufnahmen auf einem nutzerspezifischen Speicherträger gespeichert werden.

8. Mobile Datenverarbeitungsvorrichtung nach einem der Ansprüche 1 bis 5, wobei besagter Defibrillator in Form eines Sets konfiguriert ist, um eine Ausgangsbatterie des Herstellers zu ersetzen.

9. Mobile Datenverarbeitungsvorrichtung nach Anspruch 8 mit Mitteln für einen doppelten Betrieb mit besagtem Laptop oder ultramobilem Gerät:
einen ersten Betrieb im Rahmen eines im Computer vorhandenen nativen Betriebssystems zur Durchführung von unkritischen Wartungsvorgängen des Defibrillationssystems;
einen zweiten Betrieb, der das Herunterfahren des vorhandenen Betriebssystems im Computer erzwingt, um die Vollständigkeit der Ressourcen der Defibrillationsvorrichtung zu bewahren.

10. Mobile Datenverarbeitungsvorrichtung nach Anspruch 9, die einen Stecker vom Typ USB umfasst, wodurch ein Kaltstart des Computers ermöglicht wird.

11. Mobile Datenverarbeitungsvorrichtung nach Anspruch 8 und 10 mit:
- Mitteln zum Erkennen des Anschlusses von Defibrillationselektroden;
- Steuermitteln, als Reaktion auf die Ermittlung des besagten Anschlusses, um:
- das Herunterfahren des Betriebssystems als Reaktion auf die Ermittlung des Anschlusses von Defibrillationselektroden zu erzwingen;
- den USB-Anschluss zu aktivieren, um einen sofortigen Kaltstart (boot) über besagten USB-Anschluss zu gestatten.

## Claims

1. A data processing device under the form of a mobile laptop computer (390, 490, 590), a touchpad or a smartphone (10); said mobile device being configured for running a secured booting procedure using the capture of a fingerprint and further comprising :
a defibrillator device (100); a battery (150) for powering said defibrillator device; an assistance software for the use of the defibrillator by the general public;
a medical diagnostic and monitoring control unit, comprising at least one sensor allowing the monitoring of one physiological date of the user of the device, said user's physiological data consisting of one of the following: the heart rate, the tension, the temperature, the oxygen saturation pressure, PHCT, said mobile device being configured to capture and store said user's physiological data together with the execution of a booting procedure so as to allow a secure storage of said physiological data.

2. The mobile data processing device according to claim 1, comprising an infrared sensor for sensing the level of oxygen saturation and the cardiac frequency of the user of the mobile device, as well as the periodical storage within a controlled storage area by said diagnostic software.

3. The mobile data processing device according to claim 2, wherein said infrared sensor is located proximate to said fingerprint sensor being present for security purpose, so as to jointly allow:
- the sensing of the pressure and heart rate curves simultaneously with the sensing of the fingerprint serving for securing the activation of the system;
- the signing of the pressure and heart rate curves by information derived from the fingerprint sensor so as to irreversibly associate the data entered by the infrared sensor and the user's fingerprint.

4. The mobile device according to claim 3 comprising a device for monitoring the sleep apnea.

5. The mobile data processing device according to claim 3 comprising two electrodes serving for the capture of an electrocardiogram and:
- means for storing the electrocardiogram within the device;
- means for analyzing the electrocardiogram and comparing it with a reference image;
- in response to said comparison, means of alerting the user of the device

6. A monitoring process for a mobile device as defined in claim 1 to 5, comprising the following steps:
- capturing a reference ECG image, simultaneously with a user identification data;
- signing the reference image by means of the identification data of the user,
- transmitting the reference image to a server or to a remote system for validation of the reference image;
- receiving a validation confirmation of the reference image;
- storing the validated image as a reference image and using it for comparison and analysis with subsequent captures of ECG by said mobile system.

7. The monitoring process for a mobile device as defined in claim 6, wherein the subsequent ECG recordings are stored in a storage media which is specific to the user.

8. A mobile data processing device as defined in anyone of claims 1 to 5, wherein said defibrillator is configured under the form of a kit which can replace the original battery provided by the device manufacturer.

9. A mobile data processing device according to claim 8 comprising means for arranging two different modes of operation of the portable computer or laptop:
- a first mode of operation wherein the device is operated within the native operating system present within said device, for the purpose of performing non critical maintenance procedures of the defibrillator device;
- a second mode of operation wherein the device is forced to shut down the running of the operating system existing within the computer, so as to preserve all resources for the sole defibrillator device.

10. A mobile data processing device according to claim 9 comprising a USB type connecter which can be used for booting the device.

11. A mobile data processing device according to claims 8 and 10 comprising:
- means for detecting the connection of the defibrillation electrodes;
- control means, in response to the detection of said connection, for:
- forcing the shutdown of the operating system in response to the detecting of the connection of the defibrillation electrodes;
- activating the USB connecter so as to allow immediate booting via said USB connector.
